# EUROPEAN PATENT SPECIFICATION

(11) **EP 3 517 030 B1**
(45) Date of publication and mention of the grant of the patent: **08.09.2021**
(21) Application number: 19152859.5
(22) Date of filing: 21.01.2019
(51) Int. Cl.: A61B 5/00

(54) **PHYSIOLOGICAL PARAMETER MONITOR WITH A CLEAT AND AN EQUIPMENT MODULE REMOVABLY ATTACHABLE TO THE CLEAT**
MONITOR FÜR PHYSIOLOGISCHE PARAMETER MIT EINER KLAMMER UND AN DER KLAMMER LÖSBAR BEFESTIGBARES AUSRÜSTUNGSMODUL
MONITEUR DE PARAMÈTRES PHYSIOLOGIQUES COMPORTANT UN TAQUET ET MODULE D'ÉQUIPEMENT POUVANT ÊTRE FIXÉ DE FAÇON AMOVIBLE AU TAQUET

(30) Priority: 24.01.2018 US 201862621232 P
(43) Date of publication of application: 31.07.2019
(73) Proprietor: Welch Allyn, Inc., Skaneateles Falls, NY 13153-0220 (US)
(72) Inventor: SUAREZ, Carlos A, Skaneateles Falls, NY New York 13153-0220 (US); MARTIN, Scott A, Skaneateles Falls, NY New York 13153-0220 (US); WAWRO, Thaddeus J, Skaneateles Falls, NY New York 13153-0220 (US); FITCH, Timothy R, Skaneateles Falls, NY New York 13153-0220 (US)
(74) Representative: Findlay, Alice Rosemary

(56) References cited:
- US-A1- 2008 114 220
- US-A1- 2008 288 026
- US-A1- 2012 226 128

## Description

The subject matter described herein relates to medical vital signs monitors and particularly to a modular monitor having a module which is wearable by a subject (e.g. a medical patient) and a detachable module which is detachable from the wearable module. When the patient participates in activities which might be harmful to components of the detachable module, the detachable module can be detached from the wearable module while the wearable module remains in place on the patient. The detachable module can be subsequently reattached to the wearable module.

Wearable monitors for monitoring medical vital signs and other physiological parameters are advantageous because they can provide continuous monitoring of the vital signs of a subject, such as a hospital patient. Wearable monitors include an adhesive layer for securing the monitor to the patient. A caregiver presses the adhesive layer of the monitor against the patient's skin to secure the monitor to the patient's body. An example of a wearable monitor is described in US 2008/0288026 A1, in the name of KONINKLIJKE PHILIPS ELECTRONICS N.V., which describes a medical device including device electrical contacts, and a patch including a base surface, and a patch connector that establishes an electrical communication path between the base surface and medical device contacts, and a mechanical connection between the patch and the medical device when the patch connector and the medical device are engaged.

One drawback of wearable monitors is the need to remove the monitor from the patient when the patient participates in activities that could cause damage to components of the monitor. Such activities include bathing, showering, and radiological procedures. At the conclusion of the activity it is desirable to resecure the wearable monitor to the patient. However the previous act of removing the monitor may have compromised the strength of the adhesive so that the monitor will no longer adhere reliably to the patient. Even if the monitor can be successfully reapplied to the patient, it is difficult to position the monitor exactly as it had been before removal. As a result the quality and/or consistency of the monitored signals may suffer. The monitor described herein overcomes at least these shortcomings of conventional vital signs monitors.

The invention will now be further described by way of example with reference to the accompanying drawings, in which:
FIG. **1** is a schematic cross sectional elevation view showing a cleat component and an equipment module component of the physiological monitor with the equipment module separated from the cleat and also showing *A* and *B* cleat electrodes, *A* and *B* connector portions of the electrodes, and *A* and *B* connector portions of the equipment module.
FIG. **2** is a view similar to FIG. **1** showing the equipment module attached to the cleat.
FIG. **3A** is an elevation view showing the cleat of FIG. **1** with only the *A* electrode illustrated to assist the reader in identifying the features of the *A* electrode.
FIG. **3B** is an elevation view showing the cleat of FIG. **1** with only the *B* electrode illustrated to assist the reader in identifying the features of the *B* electrode.
FIG. **4** is a perspective view of the cleat of FIG. **1****.**
FIG. **5** is an illustration showing the concepts of radial (**R**) and circumferential (**C**) in the context of a noncircular geometry (**G**).

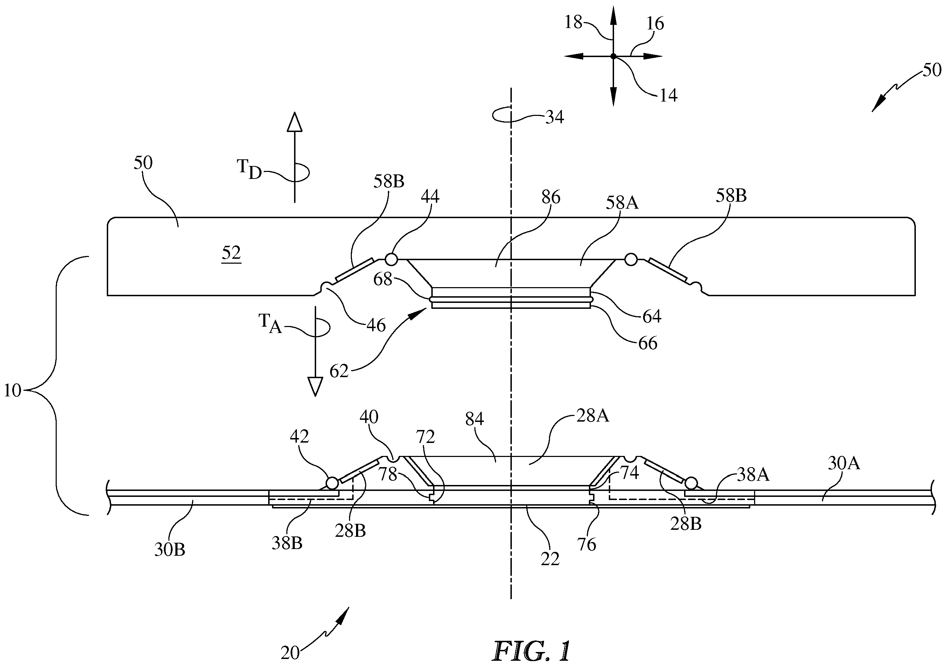

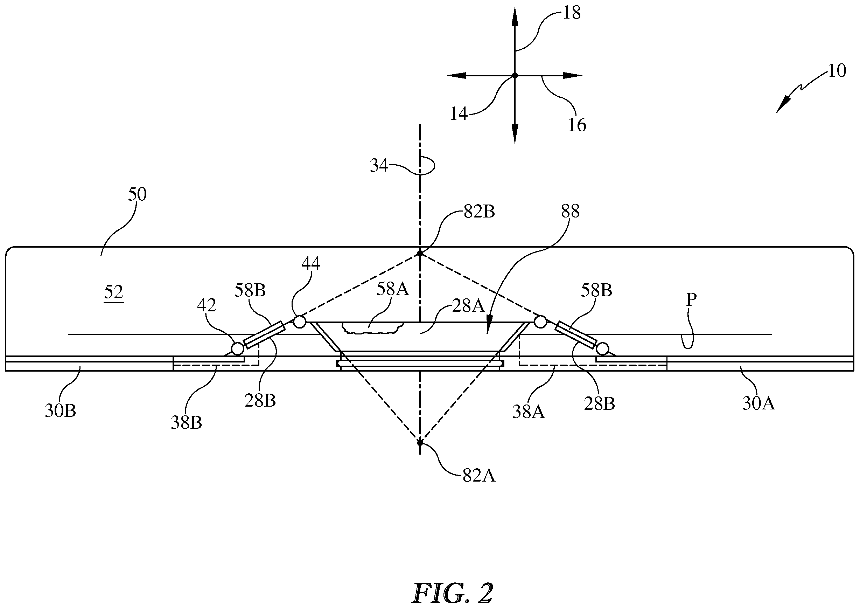

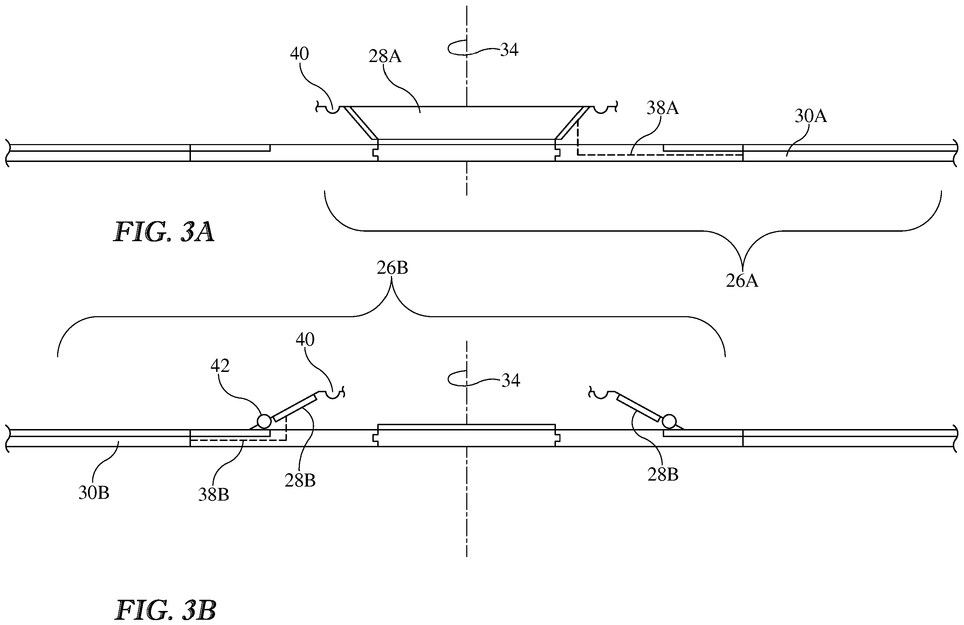

FIGS. **1** and **2** show a physiological monitor **10** in an assembled or connected state and a disassembled or disconnected state respectively and also show lateral, longitudinal, and transverse reference axes **14, 16, 18.** The monitor includes a cleat **20** adapted to be securable to a subject, such as a patient. The illustrated cleat includes an adhesive layer **22** (shown only in FIG. **1**) for securing the cleat to a patient. As seen best in FIGS. **3A** and **3B****,** the cleat includes a pair of cleat electrodes **26A, 26B** which are referred to herein as *A* and *B* electrodes. In one embodiment the electrodes are electrocardiogram (EKG) electrodes. In another embodiment the electrodes are excitation and sensing electrodes of the type employed in impedance electrocardgraphy.

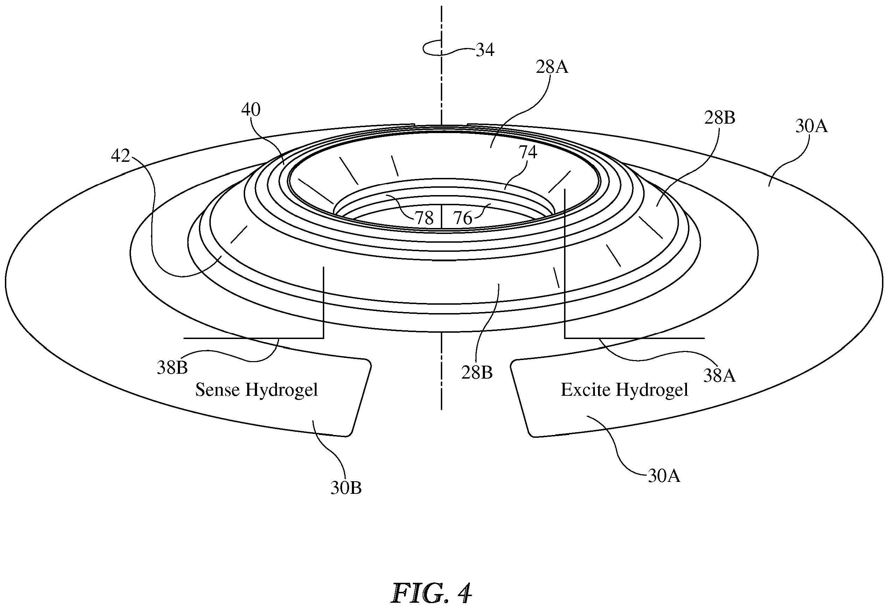

As seen best in FIGS. **3A** and **4****,** *A* electrode **26A** includes an *A* electrode connector portion **28A** and an arcuate *A* contact portion **30A.** Contact portion **30A** is the portion of the *A* electrode intended to be in contact with the patient.

Referring additionally to FIG. **3B****,** *B* electrode **26B** includes a *B* electrode connector portion **28B** and an arcuate *B* contact portion **30B.** Contact portion **30B** is the portion of the *B* electrode intended to be in contact with the patient.

In embodiments in which the electrode contact portions **30A, 30B** are EKG electrodes, the contact portions may hydrogel contact portions.

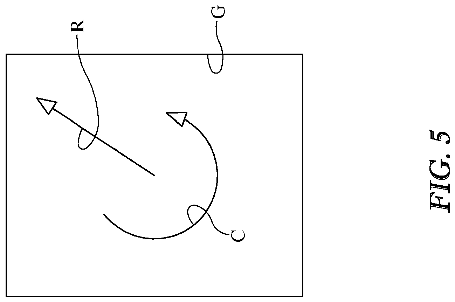

The *A* and *B* electrode connector portions **28A, 28B** are radially spaced from each other. The *A* electrode connector portion **28A** is radially inboard of the *B* electrode connector portion **28B.** The *A* electrode contact portion **30A** is radially spaced from and radially outboard of the *A* electrode connector portion **28A.** The *B* electrode contact portion **30B** is radially spaced from and radially outboard of the *B* electrode connector portion **28B.** In this specification, the use of the term "radial" and variants thereof is not intended to limit the form of any component to a circular shape, such as that seen in FIG. **3****.** Instead, "radial" is used to describe spatial relationships relative to a reference point or axis, such as axis **34.** The terms "inboard", "outboard" and their variants are used to describe locations which are respectively closer to or more distant from a reference axis or reference point. The use of "circumferential" and its variants in this specification is also not intended to limit the form of any component to a circular shape. FIG. **5** shows the concepts of radial (**R**) and circumferential (**C**) in the context of a noncircular geometry (**G**).

A cleat O-ring seat **40** resides radially between the *A* and *B* electrode connector portions **28A, 28B.** A cleat O-ring **42** resides radially between *B* connector portion **28B** and contact portions **30A, 30B.**

An A conductor element **38A** connects the *A* contact portion **30A** to the *A* connector portion **28A** in order to convey signals between contact portion **30A** and connector portion **28A.** A *B* conductor element **38B** connects the *B* contact portion **30B** to the *B* connector portion **28B** in order to convey signals between contact portion **30B** and connector portion **28B.** As illustrated, conductor elements **38A, 38B** are considerably more localized than the connector and contact portions **28A, 28B, 30A, 30B,** all of which have a substantial circumferential spread. Nevertheless, conductor elements **38A, 38B** could be more circumferentially extensive than those illustrated.

The monitor also includes an equipment module **50** removably attached to or removably attachable to the cleat as described in more detail below. Equipment module **50** includes electronic components of the monitor housed in an equipment compartment **52.** The electronic components may include at least a processor for processing data signals from electrodes **26A, 26B** and from one or more sensors other than the electrodes (described below). The compartment may also house one or more amplifiers, one or more filters to amplify and de-noise the sensor and electrode signals, and a transceiver to provides communication with remote devices such as information displays and user controls. The compartment may also house a battery. Further examples of equipment modules, cleats, and their architectures may be found in US provisional patent applications 62/588,598 filed on Nov. 20, 2017, 62/592,602 filed on Nov. 30, 2017, 62/618,772 filed on Jan. 18, 2018, and 62/607,646 filed on Dec. 19, 2017, published as US2019150739 and US2019190293.

Equipment module **50** has an *A* module connector portion **58A** and a *B* module connector portion **58B.** The *A* module connector portion **58A** is radially inboard of the *B* module connector portion **58B.** As a result the *A* and *B* module connector portions **58A, 58B** connect respectively with the *A* and *B* electrode connector portions **28A, 28B** of the cleat when the equipment module is connected to the cleat as in FIG. **2****.** In the illustrated embodiment the *A* module connector portion **58A** and the *A* electrode connector portion **28A** are adapted to mate or connect with each other by being in mutual contact. The mutual contact establishes an *A* signal connection and resultant signal communication between the cleat and the equipment module, but does not establish a mechanical connection between the cleat and the equipment module. Likewise the *B* module connector portion **58B** and the *B* electrode connector portion **28B** are adapted to connect with each other by being in mutual contact thereby establishing a *B* signal connection and resultant signal communication, but not a mechanical connection, between the cleat and the equipment module. Mechanical connection is provided by mechanical connector elements described below.

An equipment module O-ring **44** resides radially between module connector portions **58A, 58B.** An equipment module O-ring seat **46** resides radially outboard of *B* module connector portion **58B.** When the equipment module is connected to the cleat as seen in FIG. **2****,** cleat O-ring **42** fits into module O-ring seat **46,** and module O-ring **44** fits into cleat O-ring seat **40.**

Equipment module **50** includes a module mechanical connector element **62** which extends transversely from the *A* module connector portion **58A.** The illustrated connector element **62** comprises two cylindrical sections **64, 66** and an intervening ring **68** whose diameter is greater than that of the cylindrical sections.

Cleat **20** includes a cleat mechanical connector element **72** which extends transversely from *A* electrode connector portion **28A.** The illustrated connector element **72** comprises two cylindrical sections **74, 76** and an intervening groove **78** with a diameter greater than that of the cylindrical sections. The module and cleat mechanical connector elements are adapted to mate with each other to affect a mechanical connection between cleat **20** and equipment module **50.** In particular ring **68** snaps into groove **78** to affect the mechanical connection. The ring is also separable from the groove so that the equipment module can be detached from the cleat. As noted earlier in this specification, the fact that the equipment module is attachable to and detachable from cleat allows the equipment module to be temporarily removed from the cleat when the patient participates in activities that could cause damage to components of the equipment module. The equipment module can be subsequently re-attached to the cleat.

In the illustrated embodiment the mechanical connection between the cleat and the equipment module is distinct from the *A* signal connection defined by the *A* connector portions **28A, 58A** of the cleat electrodes and the equipment module and is also distinct from the *B* signal connection defined by the *B* connector portions **28B, 58B** of the cleat electrodes and the equipment module.

When the cleat and equipment module are connected to each other as seen in FIG. **2****,** The *A* and *B* electrode contact portions **30A, 30B** are radially outboard of both the electrode *A* and *B* connector portions **28A, 28B** and the *A* and *B* equipment module connector portions **58A, 58B.**

In the specific embodiment shown in the drawings the *A* and *B* electrode connector portions **28A, 28B** are circumferentially complete, i.e. they extend circumferentially 360 degrees to define an *A* signal connector ring **28A** and a *B* signal connector ring **28B** which circumscribes the *A* signal connector ring. The *A* cleat contact portion **30A** is radially spaced from and is in signal communication, by way of conductor **38A,** with the *A* cleat signal connector ring **28A.** The *B* cleat contact portion **30B** is radially spaced from and is in signal communication by way of conductor **38B,** with the *B* cleat signal connector ring **28B.** The *A* and *B* module connector portions **58A, 58B** are also complete signal connector rings arranged so that the *B* module signal connector ring **58B** circumscribes the *A* module signal connector ring **58A.**

Referring to FIG. **2****,** the *A* cleat signal connector ring **28A** and the *A* module signal connector ring **58A** have similar frustoconical profiles having a virtual vertex **82A.** The *B* cleat signal connector ring **28B** and the *B* module signal connector ring **58B** also have similar frustoconical profiles having a virtual vertex **82B.** (In the illustration the conical profiles are represented by the interface between rings **28A** and **28B** and the interface between rings **58A** and **58B,** hence the presence of only a single apex on each side of the monitor.) "Similar" is used in the geometric sense of having the same shape. When the equipment module is connected to the cleat the apexes **82A, 82B** are transversely separated from each other on opposite sides of center plane **P.** The A module signal connector ring **58A** nests radially inside the *A* cleat signal connector ring **28A,** and the *B* cleat signal connector ring **28B** nests radially inside the *B* module connector ring **58B.**

As seen in FIG. **1****,** cleat **20** includes a cleat sensor opening **84** radially inboard of cleat *A* electrode connector portion **28A.** Equipment module **50** includes a module sensor opening **86** radially inboard of the *A* module connector portion **58A.** When the cleat is assembled to the module as seen in FIG. **2****,** connector portions **84, 86** define a monitor sensor opening **88.** In the specific embodiment in which the *A* module connector portion is a full ring **58A,** the ring is the border of opening **88.** The sensor opening is provided to accommodate sensors other than the electrodes **26A, 26B.** Examples of such sensors include photoplethysmogram (PPG) sensors, phonocardiogram (PCG) sensors, and oxygen saturation (SpO2) sensors.

The monitor is in the assembled state of FIG. 2 when it is being used to monitor the physiological signs of a patient. The cleat and equipment module are in signal communication with each other by way of an *A* signal connection established by the *A* electrode connector portion **28A** and the *A* module connector portion **58A** and by the *B* electrode connector portion **28B** and the *B* module connector portion **58B.** The equipment module **50** is detachable from the cleat **20** in the transverse direction, as indicated by arrow **T_{D}** in FIG. **1****,** so that the patient may participate in activities which might be harmful to components of the detachable equipment module. When the activity is finished the equipment module can be reconnected to the cleat in direction **T_{A}** to reproduce the assembled state of FIG. **1****.**

Although this disclosure refers to specific embodiments, it will be understood by those skilled in the art that various changes in form and detail may be made

## Claims

1. A physiological monitor (10) comprising:
a cleat (20) adapted to be securable to a subject, the cleat (20) having an *A* electrode which includes an *A* electrode connector portion (28A) and a *B* electrode which includes a *B* electrode connector portion (28B), which circumscribes the A electrode connector portion (28A);
an equipment module (50) removably attached or removably attachable to the cleat (20) in a transverse direction, the module (50) having an *A* module connector portion (58A) and a *B* module connector portion (58B) arranged such that the *A* and *B* module connector portions (58A, 58B) connect with the *A* and *B* electrode connector portions (28A, 28B) respectively when the equipment module (50) is connected to the cleat (20).

2. The monitor (10) of claim **1** wherein the *A* and *B* electrodes each include respective *A* and *B* contact portions (30A, 30B), adapted to contact the subject.

3. The monitor (10) of either claim **1** or claim **2** wherein the *A* and *B* electrodes are *A* and *B* EKG electrodes.

4. The monitor (10) of claim **3** wherein one of the *A* and *B* electrodes is an excite electrode and the other is a sense electrode.

5. The monitor (10) of any preceding claim wherein the *A* and *B* electrode connector portions (28A, 28B) are circumferentially complete.

6. The monitor (10) of claim **5** wherein the cleat (20) includes a sensor opening (84) radially inboard of the *A* electrode connector portion (28A).

7. The monitor (10) of claim **6** wherein the *A* electrode connector portion (28A) is a ring.

8. The monitor (10) of any preceding claim wherein:
the *A* electrode connector portion (28A) and the *A* module connector portion (58A) define an *A* signal connection between the cleat (20) and the module (50);
the *B* electrode connector portion (28B) and the *B* module connector portion (58B) define a *B* signal connection between the cleat (20) and the module (50);
the cleat (20) includes a cleat (20) mechanical connector element (72);
the equipment module (50) includes a module mechanical connector element (62); and
the cleat (20) and module mechanical connector elements (62, 72) are adapted to mate with each other to effect a mechanical connection between the cleat (20) and the equipment module (50) which is distinct from the signal connections.

9. The physiological monitor (10) of any preceding claim wherein:
the *A* electrode includes an *A* contact portion (30A) which is radially outboard of the *A* electrode connector portion (28A); and
the *B* electrode includes a *B* contact portion (30B) which is radially outboard of the *B* electrode connector portion (28B).

10. The monitor (10) of claim **9** wherein when the A and B contact portions (30A, 30B) each have an arcuate profile.

11. The monitor (10) of claim **10** wherein when the cleat (20) and module are connected to each other the *A* and *B* electrode contact portions (30A, 30B) are radially outboard of both the electrode *A* and *B* connector portions (28A, 28B) and the *A* and *B* equipment module connector portions (58A, 58B).

12. The monitor (10) of any one of claims **9** to **11** wherein:
the *A* electrode connector portion (28A) mates with the *A* module connector portion (58A) to define an *A* signal connection between the cleat (20) and the module (50);
the *B* electrode connector portion (28B) mates with the *B* module connector portion (58B) to define a *B* signal connection between the cleat (20) and the module (50);
the cleat (20) includes a cleat (20) mechanical connector element (72);
the equipment module (50) includes a module mechanical connector element (62); and
the cleat (20) and module mechanical connector elements (62, 72) are adapted to mate with each other to effect a mechanical connection between the cleat (20) and the equipment module (50) which is distinct from the signal connections.

13. The monitor (10) of any one of claims **9** to **12** wherein the *A* electrode connector portion (28A) and the *A* module connector portion (58A) are rings having similar frustoconical profiles and the *B* electrode connector portion (28B) and the *B* module connector portion (58B) are also rings having similar frustoconical profiles.

14. The monitor (10) of claim **13** wherein:
the similar frustoconical profiles of the *A* connector rings have *A* apexes;
the similar frustoconical profiles of the *B* connector rings have *B* apexes; and
when the cleat (20) and module (50) are connected to each other the *A* apexes lie on one side of a laterally and longitudinally extending centerplane and the *B* apexes lie on the other side of the laterally and longitudinally extending center plane.

15. The monitor (10) of claim **14** wherein when the cleat (20) and the module (50) are connected to each other:
the *A* module connector ring nests radially inside the *A* cleat (20) electrode connector ring; and
the *B* electrode connector ring nests radially inside the *B* module connector ring.

## Patentansprüche

1. Ein physiologischer Monitor (10), umfassend:
eine Klammer (20), die so angepasst ist, dass sie an einer Person befestigt werden kann, wobei die Klammer (20) eine *A*-Elektrode, die einen *A*-Elektroden-Verbindungsteil (28A) beinhaltet, und eine *B*-Elektrode aufweist, die einen *B*-Elektroden-Verbindungsteil (28B) beinhaltet, der den A-Elektroden-Verbindungsteil (28A) begrenzt;
ein Ausrüstungsmodul (50), das an der Klammer (20) in einer Querrichtung lösbar befestigt oder lösbar befestigbar ist, wobei das Modul (50) einen A-Modul-Verbindungsteil (58A) und einen B-Modul-Verbindungsteil (58B) aufweist, die so angeordnet sind, dass die A- und B-Modul-Verbindungsteile (58A, 58B) mit den A- bzw. B-Elektroden-Verbindungsteilen (28A, 28B) verbunden sind, wenn das Ausrüstungsmodul (50) mit der Klammer (20) verbunden ist.

2. Der Monitor (10) nach Anspruch 1, wobei die A- und die B-Elektrode jeweils entsprechende A- und B-Kontaktteile (30A, 30B) beinhalten, die so angepasst sind, dass sie die Person berühren.

3. Der Monitor (10) nach entweder Anspruch 1 oder Anspruch 2, wobei die A- und B-Elektroden A- und B-EKG-Elektroden sind.

4. Der Monitor (10) nach Anspruch 3, wobei eine der A- und B-Elektroden eine Erregungselektrode und die andere eine Detektionselektrode ist.

5. Der Monitor (10) nach einem der vorstehenden Ansprüche, wobei die A- und B-Elektroden-Verbindungsteile (28A, 28B) vollständig rund herum verlaufen.

6. Der Monitor (10) nach Anspruch 5, wobei die Klammer (20) eine Sensoröffnung (84) beinhaltet, die radial innerhalb des A-Elektroden-Verbindungsteils (28A) verläuft.

7. Der Monitor (10) nach Anspruch 6, wobei der A-Elektroden-Verbindungsteil (28A) ein Ring ist.

8. Der Monitor (10) nach einem der vorstehenden Ansprüche, wobei:
der A-Elektroden-Verbindungsteil (28A) und der A-Modul-Verbindungsteil (58A) eine A-Signal-Verbindung zwischen der Klammer (20) und dem Modul (50) definieren;
der B-Elektroden-Verbindungsteil (28B) und der B-Modul-Verbindungsteil (58B) eine B-Signal-Verbindung zwischen der Klammer (20) und dem Modul (50) definieren;
die Klammer (20) ein mechanisches Verbindungselement (72) für die Klammer (20) beinhaltet;
das Ausrüstungsmodul (50) ein mechanisches Verbindungselement (62) für das Modul beinhaltet; und
die mechanischen Verbindungselemente (62, 72) für die Klammer (20) und das Modul so angepasst sind, dass sie sich zusammenfügen, um eine mechanische Verbindung zwischen der Klammer (20) und dem Ausrüstungsmodul (50) zu bewirken, die sich von den Signalverbindungen unterscheidet.

9. Der physiologische Monitor (10) nach einem der vorstehenden Ansprüche, wobei:
die A-Elektrode einen A-Kontaktteil (30A) beinhaltet, der radial außerhalb des A-Elektroden-Verbindungsteils (28A) verläuft; und
die B-Elektrode einen B-Kontaktteil (30B) beinhaltet, der radial außerhalb des B-Elektroden-Verbindungsteils (28B) verläuft.

10. Der Monitor (10) nach Anspruch 9, wobei die A- und B-Kontaktteile (30A, 30B) jeweils ein gebogenes Profil aufweisen.

11. Der Monitor (10) nach Anspruch 10, wobei, wenn die Klammer (20) und das Modul miteinander verbunden sind, die A- und B-Elektroden-Kontaktteile (30A, 30B) radial sowohl außerhalb der A- und B-Elektroden-Verbindungsteile (28A, 28B) als auch der A- und B-Ausrüstungsmodul-Verbindungsteile (58A, 58B) verlaufen.

12. Der Monitor (10) nach einem der Ansprüche 9 bis 11, wobei:
sich der A-Elektroden-Verbindungsteil (28A) mit dem A-Modul-Verbindungsteil (58A) zusammenfügt, um eine A-Signal-Verbindung zwischen der Klammer (20) und dem Modul (50) zu definieren;
sich der B-Elektroden-Verbindungsteil (28B) mit dem B-Modul-Verbindungsteil (58B) zusammenfügt, um eine B-Signal-Verbindung zwischen der Klammer (20) und dem Modul (50) zu definieren;
die Klammer (20) ein mechanisches Verbindungselement (72) für die Klammer (20) beinhaltet;
das Ausrüstungsmodul (50) ein mechanisches Verbindungselement (62) für das Modul beinhaltet; und
die mechanischen Verbindungselemente (62, 72) für die Klammer (20) und das Modul so angepasst sind, dass sie sich zusammenfügen, um eine mechanische Verbindung zwischen der Klammer (20) und dem Ausrüstungsmodul (50) zu bewirken, die sich von den Signalverbindungen unterscheidet.

13. Der Monitor (10) nach einem der Ansprüche 9 bis 12, wobei der A-Elektroden-Verbindungsteil (28A) und der A-Modul-Verbindungsteil (58A) Ringe mit ähnlichen kegelstumpfförmigen Profilen sind und der B-Elektroden-Verbindungsteil (28B) und der B-Modul-Verbindungsteil (58B) ebenfalls Ringe mit ähnlichen kegelstumpfförmigen Profilen sind.

14. Der Monitor (10) nach Anspruch 13, wobei:
die ähnlichen kegelstumpfförmigen Profile der A-Verbindungsringe A-Scheitelpunkte aufweisen,
die ähnlichen kegelstumpfförmigen Profile der B-Verbindungsringe B-Scheitelpunkte aufweisen; und
wenn die Klammer (20) und das Modul (50) miteinander verbunden sind, die A-Scheitelpunkte auf einer Seite einer sich seitlich und längs erstreckenden Mittelebene und die B-Scheitelpunkte auf der anderen Seite einer sich seitlich und längs erstreckenden Mittelebene liegen.

15. Der Monitor (10) nach Anspruch 14, wobei, wenn die Klammer (20) und das Modul (50) miteinander verbunden sind:
der A-Modul-Verbindungsring radial im Inneren des Elektroden-Verbindungsrings der A-Klammer (20) liegt; und
der B-Elektroden-Verbindungsring radial im Inneren des B-Modul-Verbindungsrings liegt.

## Revendications

1. Moniteur (10) physiologique comprenant :
un taquet (20) conçu pour être attaché à un sujet, le taquet (20) comportant une électrode *A* qui comprend une partie connecteur (28A) d'électrode *A* et une électrode *B* qui comprend une partie connecteur (28B) d'électrode *B,* qui encercle la partie connecteur (28A) d'électrode A ;
un module (50) d'équipement fixé de façon amovible ou pouvant être fixé de façon amovible au taquet (20) dans le sens transversal, le module (50) comportant une partie connecteur (58A) de module A et une partie connecteur (58B) de module B disposé de manière à ce que les parties connecteur (58A, 58B) de module A et B se connectent aux parties connecteur (28A, 28B) d'électrodes A et B respectivement lorsque le module (50) d'équipement est relié au taquet (20).

2. Moniteur (10) selon la revendication 1, dans lequel les électrodes A et B comprennent chacune les parties de contact A et B (30A, 30B) respectives, conçues pour entrer en contact avec le sujet.

3. Moniteur (10) selon soit la revendication 1, soit la revendication 2, dans lequel les électrodes A et B sont des électrodes d'ECG.

4. Moniteur (10) selon la revendication 3, dans lequel l'une des électrodes A et B est une électrode d'excitation et l'autre est une électrode de détection.

5. Moniteur (10) selon l'une quelconque des revendications précédentes, dans lequel les parties connecteur (28A, 28B) d'électrode A et B sont complètes en circonférence.

6. Moniteur (10) selon la revendication 5, dans lequel le taquet (20) comprend une ouverture de capteur (84) radialement à l'intérieur de la partie connecteur (28A) d'électrode A.

7. Moniteur (10) selon la revendication 6, dans lequel la partie connecteur (28A) d'électrode A est une bague.

8. Moniteur (10) selon l'une quelconque des revendications précédentes, dans lequel :
la partie connecteur (28A) d'électrode A et la partie connecteur (58A) de module A délimitent une connexion de signal A entre le taquet (20) et le module (50) ;
la partie connecteur (28B) d'électrode B et la partie connecteur (58B) de module B délimitent une connexion de signal B entre le taquet (20) et le module (50) ;
le taquet (20) comprend un élément de liaison mécanique (72) de taquet (20) ;
le module (50) d'équipement comprend un élément de liaison mécanique (62) de module ; et
le taquet (20) et les éléments de liaison mécanique (62, 72) de module sont conçus pour s'accoupler les uns aux autres afin d'établir une liaison mécanique entre le taquet (20) et le module (50) d'équipement, qui est distincte des connexions de signal.

9. Moniteur (10) physiologique selon l'une quelconque des revendications précédentes dans lequel :
l'électrode A comprend une partie de contact A (30A) qui est radialement à l'extérieur de la partie connecteur (28A) d'électrode A ; et
l'électrode B comprend une partie de contact B (30B) qui est radialement à l'extérieur de la partie connecteur (28B) d'électrode B.

10. Moniteur (10) selon la revendication 9, dans lequel les parties de contact A et B (30A, 30B) possèdent chacune un profil arqué.

11. Moniteur (10) selon la revendication 10, dans lequel lorsque le taquet (20) et le module sont connectés l'un à l'autre, les parties de contact (30A, 30B) des électrodes A et B sont radialement à l'extérieur des parties connecteur (28A, 28B) des électrodes A et B et des parties connecteur de module d'équipement A et B (58A, 58B).

12. Moniteur (10) selon l'une quelconque des revendications 9 à 11, dans lequel :
la partie connecteur (28A) d'électrode A s'accouple avec la partie connecteur (58A) de module A pour délimiter une connexion de signal A entre le taquet (20) et le module (50) ;
la partie connecteur (28B) d'électrode B s'accouple avec la partie connecteur (58B) de module B pour délimiter une connexion de signal B entre le taquet (20) et le module (50) ;
le taquet (20) comprend un élément de liaison mécanique (72) de taquet (20) ;
le module (50) d'équipement comprend un élément de liaison mécanique (62) de module ; et
le taquet (20) et les éléments de liaison mécanique (62, 72) de module sont conçus pour s'accoupler les uns aux autres afin d'établir une liaison mécanique entre le taquet (20) et le module (50) d'équipement, qui est distincte des connexions de signal.

13. Moniteur (10) selon l'une quelconque des revendications 9 à 12, dans lequel la partie connecteur (28A) d'électrode A et la partie connecteur (58A) de module A sont des bagues présentant des profils tronconiques similaires et la partie connecteur (28B) d'électrode B et la partie connecteur (58B) de module B sont également des bagues présentant des profils tronconiques similaires.

14. Moniteur (10) selon la revendication 13, dans lequel :
les profils tronconiques similaires des bagues de connecteur A possèdent des pointes A ;
les profils tronconiques similaires des bagues de connecteur B possèdent des pointes B ; et
lorsque le taquet (20) et le module (50) sont reliés l'un à l'autre, les pointes A se trouvent d'un côté du plan central s'étendant latéralement et longitudinalement, et les pointes B se trouvent de l'autre côté du plan central s'étendant latéralement et longitudinalement.

15. Moniteur (10) selon la revendication 14, dans lequel lorsque le taquet (20) et le module (50) sont reliés l'un à l'autre :
la bague de connecteur de module A s'emboîte radialement dans la bague de connecteur d'électrode A de taquets (20) ; et
la bague de connecteur d'électrode B s'emboîte radialement dans la bague de connecteur de module B.
